# EUROPEAN PATENT APPLICATION

(11) **EP 1 593 744 A1**
(43) Date of publication of application: **09.11.2005**
(21) Application number: 03815507.3
(22) Date of filing: 27.01.2003
(51) Int. Cl.: C12Q 1/68

(54) **THE METHOD OF DETECTING THE CACNA1H MUTANT GENE OF CHILDHOOD ABSENCE EPILEPSY-THE MAIN FUNCTION GENE, AND THE CACNA1H MUTANT GENE**

(71) Applicant: Sinogenomax Co., Ltd., Beijing 100176 (CN)
(72) Inventor: WU, Xiru, Beijing 100034 (CN); SHEN, Yan, Beijing 100176 (CN); CHEN, Yucai, Beijing 100034 (CN); WU, Husheng, Beijing 100045 (CN); XU, Keming; Capital Institute of Pediatrics, Beijing 100020 (CN); PAN, Hong, Beijing 100034 (CN); ZHANG, Yuehua, Beijing 100034 (CN); LIU, Xiaoyan, Beijing 100034 (CN); LV, Jianjun, Beijing 100034 (CN); JIANG, Yuwu, Beijing 100034 (CN)
(74) Representative: Webber, Philip Michael
(86) International application number: PCT/CN2003/000087
(87) International publication number: WO 2004/067769

(57) **Abstract**

The present invention relates to a method of detecting the CACNA1H mutant gene of childhood absence epilepsy-the main function gene, the said method is directly sequencing or restriction analysis. The present invention relates to CACNA1H mutant gene. The present invention further relates to the use of the said detection and mutant gene. The present invention connects the CACNA1H gene with medicine for treating childhood absence epilepsy, proving new target site for medicine for treating the same. The present invention establishes the foundation for developing new medicines for treating childhood absence epilepsy and other type of idiopathic system epilepsy as well as other system diseases associated with CACNA1H gene.

## Description

### FIELD OF THE INVENTION

The present invention relates to a method for detecting the mutant genes resulting in childhood absence epilepsy (CAE) and the mutant genes, more particularly the present invention relates to a method for detecting the mutant genes of CACNA1H which is one of the major genes relating to CAE. This invention further relates to a kit for detecting the mutant genes mentioned above and its application. In addition, this invention also relates to the application of above detection method and the mutant genes in examination and/or therapy method of CAE.

### BACKGROUND OF THE INVENTION

Childhood absence epilepsy (CAE) is a kind of general idiopathic epilepsy, roughly accounting for 5%-15% of total childhood epilepsy with girl patients more than boy. CAE usually attack between age 3-12 with peak age at 6 to 7. The clinical symptoms include sudden unconsciousness without tumble, both eyes gazing, stopping action at his (hers) initial state. These symptoms last for few seconds to1-2 minutes. After that consciousness is recovered and continues his (her) original action without lethargy or trance. However, attack is as frequent as several even over hundred times each day. When CAE breakout electroencephalogram of the patient presents bilateral symmetric diffuse type, synchronous high echo 3Hz slow sharp wave lasting about 10 minutes, which is quicker as 3.5-4Hz at beginning, 3Hz in the middle and may slow down to 2.5-2Hz by the end, while the background wave is normal. Hyperventilation will induce CAB attacking. Frequent attack of CAE would affect study. If it is not controlled at early time or stop medication too early the general tonic spasm will be complicated in 40% of CAE. It would seriously affect sufferer's study and life and bring heavy economic burden to his (her) family and society. CAB is the common type of general idiopathic epilepsy with obvious hereditary tendency. At present it is considered as a kind of complicated hereditable disease. The pathogenesis is rather complicated and the pathogenic gene is unclear.

The T type Calcium channel is different from other types of Calcium channel in nucleotide sequence, but similar with them in structure. As other type Calcium channel it also consists of 4 repeated domains (I-IV) each consisting of 6 transmembrane regions (S1-6), and functions with the characters of transient, liable and being activated easily by low voltage. It plays a key role in the production of thalamocortical rhythm oscillation.

Up to date 3 gene families encoding T type Calcium channel have been cloned, which are CACNA1G, CACNA1H and CACNA1I. CACNA1H was mapped on chromosome 16P13.3, which encodes subunit of alpha 1h and consists of 35 exons. CACNA1H gene is extensively expressed in heart, brain and kidney. As one of the member of gene family encoding T- type Calcium channel, although CACNA1H gene (Genbank AF051946) has been cloned, yet the relationship between its function and CAE attack has not been reported and the report of studying CAE through CACNA1H gene has not been found till now.

### DISCLOSURE OF THE INVENTATION

The inventor selected T-type Calcium channel gene CACNA1H from numerous candidates as research object. The direct DNA sequencing of 35 exons of CACNA1H genes derived from 118 of child patients, Han nationality, of north China were conducted By a large number of experiments and statistical analysis of a great quantity of specimens, 12 different sites occurring missense mutation were found in 14 CAE patients, wherein two mutation sites occurred in transmembrane region and six in high conservative region, thereby the mutational CACNA1H genes were further acquired. Among the mutation sites described above, there were two mutation sites, both of which being found in each of two cases, while the mutations were not found from 230 of healthy controls. The results indicated that these mutations were not benign multiformity. Thus, based on this invention, one of the major genes relating to CAE was found.

The present invention provides a method for detecting CACNA1H mutant gene, one of the major genes relating to CAE. The method is direct DNA sequencing or restriction digestion.

The present invention also provides the CACNA1H mutant gene, one of the major genes relating to CAE.

The present invention further provides a kit used for detecting CACNA1H mutant gene, one of the major genes relating to CAE, and provides the application of the kit in examination and/or treatment method of CAE.

Furthermore, the present invention also provides the application of the detection method and mutant genes described above in examination and/or treatment method of CAE.

According to one aspect the present invention provides a method for detecting the mutation of CACNA1H gene, one of the major genes relating to CAE, that is the direct DNA sequencing or restriction enzyme digestion.

The method of direct DNA sequencing is preferred, especially, it includes following steps:
A: design primers in accordance with 35 exons of CACNA1H gene and amplify by PCR.
B: purify the PCR product and determine the DNA sequence directly. Compare the sequence data determined with that published in Genbank to identify mutation site in CACNA1H gene.
   As described in example 3 the mutation of gene can be determined directly on the gene level, and the mutation can further be determined on the protein level by translating the sequence according to the normal reading frame. Thus following step will be included.
C: Translate according to the normal reading frame thereby to confirm the mutation site in CACNA1H gene.

On the other hand the present invention provides another method for detecting the mutation site of CACNA1H gene, one of the major genes relating to CAB. The method includes following steps:
A: Isolate DNA and design primers that can anneal to some area containing the mutation site and amplify by PCR. B: Purify the PCR product and determine the DNA sequence directly. Compare the sequence data determined with that published in Genbank to identify the mutation site in CACNA1H gene. As described in example 3 the mutation of gene can be determined directly on the gene level, and the mutation can further be determined on the protein level by translating the sequence according to the normal reading frame. Thus following step will be included.
C: Translate according to the normal reading frame thereby to confirm the mutation site in CACNA1H gene.

Preferably, the mutation sites are located at the transmembrane region 2 and 3 of domain I (IS2-IS3), the transmembrane region2 of domain II (IIS2), linker between domain I and domain II (linker I-II), the join site between transmembraneregion S5 of domain I and core region (IS5-SS1), or the join site between transmembrane region S 5 of domain III and core region (IIIS5-SS1).More preferably, the nucleotide mutation sites are at least located at the sites selected from the group consisting of C562A (corresponding amino acid mutation F161L), G923A (corresponding amino acid mutation E282K), T1445A (corresponding amino acid mutation C456S), G1574A (corresponding amino acid mutation G499S), C2022T (corresponding amino acid mutation P648L), G2310A (corresponding amino acid mutation R744Q), C2322T (corresponding amino acid mutation A748V), G2397A (corresponding amino acid mutation G773D), G2429A (corresponding amino acid mutation G784S), G2570A (corresponding amino acid mutation V831M), G2621A (corresponding amino acid mutation G84SS), G4466A (corresponding amino acid mutation D1463N). Most preferably, the mutation sites are C562A (corresponding amino acid mutation F161L) locating at transmembrane region2 and 3 of domain I (IS2-IS3), G923A (corresponding amino acid mutation E282K) locating at join site between transmembrane region S5 of domain I and core region (IS5-SS1), G2570A (corresponding amino acid mutation V831M) and G2621A (corresponding amino acid mutation G848S) locating at transmembrane region2 of domain II (IIS), T1445A (corresponding amino acid mutation C456S) locating at linker of domain I and II (linker I-II) and G4466A (corresponding amino acid mutation D1463N) locating at join site between transmembrane region S5 of domain III and core region (IIIS5-SS1).

According to another aspect of this present invention 12 missenes mutation sites were found from CAE patients (as shown in table 3) through direct DNA sequencing method specified by this invention. Among these mutation sites two occurred in transmembrane region and six in high conservative region. Thereby the mutational CACNA1H genes were further acquired. Both mutation sites R744Q and G773D appeared in two different CAE patients while the mutation sites mentioned above were not found from 230 of healthy controlls. The results indicated that these mutations were not benign multiformity. Thus, based on this invention the CACNA1H mutant gene, one of the major genes relating to CAE was found. The mutant gene contains DNA sequence of T-type Calcium channel gene CACNA1H and carries at least one mutation site locating at the site selected from the group consisting of the transmembrane region 2 and 3 of domain I (IS2-IS3), the join site between transmembrane region S5 of domain I and core region (IS5-SS1), the transmembrane region2 of domain II (IIS2), linker between domain I and domain II (linkerI-II), or the join site between transmembrane region S5 of domain III and core region (IIIS5 -SS 1). Preferably at least one of the mutation sites is located at one of the following sites in CACNA1H sequence: C562A (corresponding amino acid mutation F161L), G923A (corresponding amino acid mutation E282K), T1445A (corresponding amino acid mutation C456S), G1574A (corresponding amino acid mutation G499S), C2022T (corresponding amino acid mutation P648L), G2310A (corresponding amino acid mutation R744Q), C2322T (corresponding amino acid mutation A748V), G2397A (corresponding amino acid mutation G773D), G2429A (corresponding amino acid mutation G784S), G2570A (corresponding amino acid mutation V831M), G2621A (corresponding amino acid mutation G848S), G4466A (corresponding amino acid mutation D1463N). More preferably at least one of the mutation sites is the site selected from the group consisting of C562A, (corresponding amino acid mutation F161L) locating at transmembrane region2 and 3 of domain I (IS2-IS3), G923A (corresponding amino acid mutation E282K) locating at join site between transmembrane region S5 of domain I and core region (IS5-SS1), G2570A (corresponding amino acid mutation V831M) and G2621A (corresponding amino acid mutation G848S) locating at transmembrane region2 of domain II (IIS), T1445A (corresponding amino acid mutation C456S) locating at linker of domain I and II (linker I-II) and G4466A (corresponding amino acid mutation D1463N) locating at join site between transmembrane region S5 of domain III and core region (IIIS5- S51).

This present invention also provides a kit for detecting mutant genes of CACNA1H, which is one of the major genes relating to CAE. The Kit consists of one or several vessels containing one or several components used for detection of CACNA1H mutant genes. The different components could be included in the kit depending on the detection method used and the sites to be detected Provided in conjunction with the kit is the information about the manufacture, use and sale of medicines and biologics which have been examined and verified by the government drug administration. For example, the kit which is used to direct detection of mutation sites of CACNA1H in samples obtained from PCR amplification (see example 1) can contain the primers for amplification as shown in table 1, dNTP, one or several kinds of DNA polymerase and buffer solution thereof for PCR. It is known to the skilled in the art, above components are only sketchy, such as the primers mentioned can be designed in accordance with the known nucleotide sequence. They usually contain 15-30 bases with GC content of 45-50% and anneal specially to the template under the proper temperature. The primers can be designed using special computer program, (for example OLIGO 4.06 primer analysis software, Primer 3 software). The DNA polymerase used for PCR can be the enzymes of Taq DNA polymerase, such as Hotstar Taq enzyme, Klenow fragment, Tth DNA polymerase, VENT DNA polymerase etc. , which can be used for PCR amplification.
The method for direct DNA sequencing is further illustrated as follows:
1) PCR amplification
   A total of 3 5 pairs of PCR primers (table 1) are designed in accordance with the 35 exons of CACNA1H gene using software primer 3. PCR protocol: For the first 15 circles ,denature at 94°C for 30 sec, anneal at 63°C for 60 sec with annealing temperature decrease progressively by 0.5°C each circle, extended at 72°C for 110 sec. For later 25 cycles , denature at 94°C for 30 sec, anneal at 56°C for 30 sec , extend at 72°C for 40 sec. Finally extend at 72°C for 10 min.
2) Purification of PCR product
   Draw air from Multiscreen-PCR plate containing PCR product, add deioning water, stand for a while, put Multiscreen-PCR plate on mixer and shake it, redissolve the purified PCR product and place it into another clean 96-well plate.
3) Sequencing reaction and verification.
   Sequencing reaction is performed on PerkElner 9700 thermocycler with one primer of the pair of PCR primers as sequencing primer. After reaction extension products are applied to AB 1 PRISM 3700 DNA analyzer. Analyzing the sequence map obtained, appearance of hybrid peak at some site indicated that this site is in a hybrid state and needs to be further verified. First, sequencing reaction is performed again but using the opposite primer of the pair of PCR primers as sequencing primer. If the result confirms that it is really a mutation site, then the sequencing of DNA fragment containing this site, which is taken from the parents of the child patient, is performed. Our results showed that all the mutations found could appear in one of the parents, so they were the hybrid mutations. Afterwards the results of sequencing reaction of CACNA1H gene taken from the parents of 230 healthy controls revealed that no any mutation site could be found. The kit can detect the mutant genes of CACNA1H, the major gene relating to CAE, simply, handily and quickly. Therefore it is suitable to apply to the examination and treatment method of CAE.

In addition, the primers can be designed directly against a certain or some mutation sites. For example, the kit can include one or several pairs of primers shown in table 1, dNTP, and one or more kinds of DNA polymerase and buffer solutions thereof for PCR. Thus a special kit containing primers for the specified mutation sites and used for PCR amplification, product purification and sequencing is prepared

The kit of this invention can also be prepared by using restriction enzymes. For example, the kit can includes: 1) primers for amplifying the mutation sites 2) enzyme and corresponding buffer solutions for PCR amplification 3) restriction enzymes corresponding with digestion of different mutation sites 4) Restriction map of DNA fragment containing mutation sites.

It is known to the general skilled in the art that for the detection of different mutation sites the primers and restriction enzymes should be specifically designed taken aim at being suitable to the site to be detected The detection includes the following steps: PCR amplification of blood DNA taken from patient, then digesting the PCR product with restriction enzymes and comparing the digestion map obtained with restriction map provided with kit.

This present invention also provides the application of the detection method of CACNA1H mutant genes in examination and /or treatment method of CAE, and the application of CACNA1H mutant genes in examination and/or treatment method of CAE. For example, according to the invention CACNA1H gene has been confirmed a major gene relating to CAE, furthermore the mutations especially concentrate at the sites between liker I-II, thereby the relation between CACNA1H gene and drug therapy of CAE has been founded. Thus this invention provides a new target for drug therapy of this disease. Hereafter the medicine research for CAE therapy can be carried out through such as finding some factors specially acting at these mutation sites. This invention also provides the theoretical basis of developing the new drug for CAE therapy.

Finally the present invention opens the new thinking for research on the pathogeny and its susceptible genes of other types of idiopathic epilepsy. For example mutations of other sites in CACNA1H gene may cause the attack of other types of idiopathic epilepsy. In fact the mutation of high expressed genes on the cycle route of thalamocortical are all possible to cause the attack of idiopathic epilepsy.

### BRIEF DESCRIPTION OF THE DRAWINGS

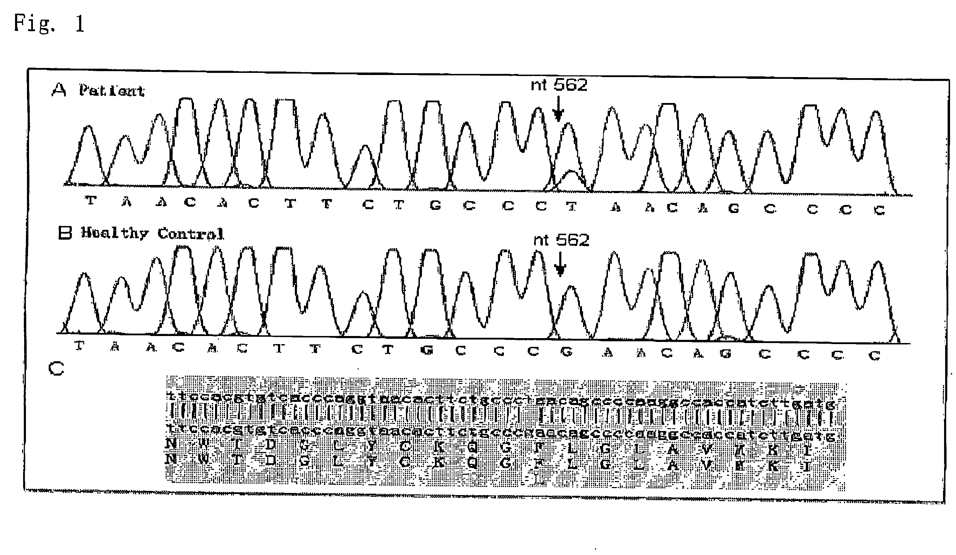

Figure 1 shows the sequence maps of partial DNA containing mutation site C562A (corresponding amino acid mutation F161L) of CACNA1H gene from CAE patient and from healthy control. A: sequence map of partial DNA containing mutation site from CAE patient. B: sequence map of partial DNA from healthy control. C: partial nucleotide sequence and its corresponding amino acid sequence containing mutation site F161L. Herein nt indicates the position of mutant nucleotide in sequence AF051946.

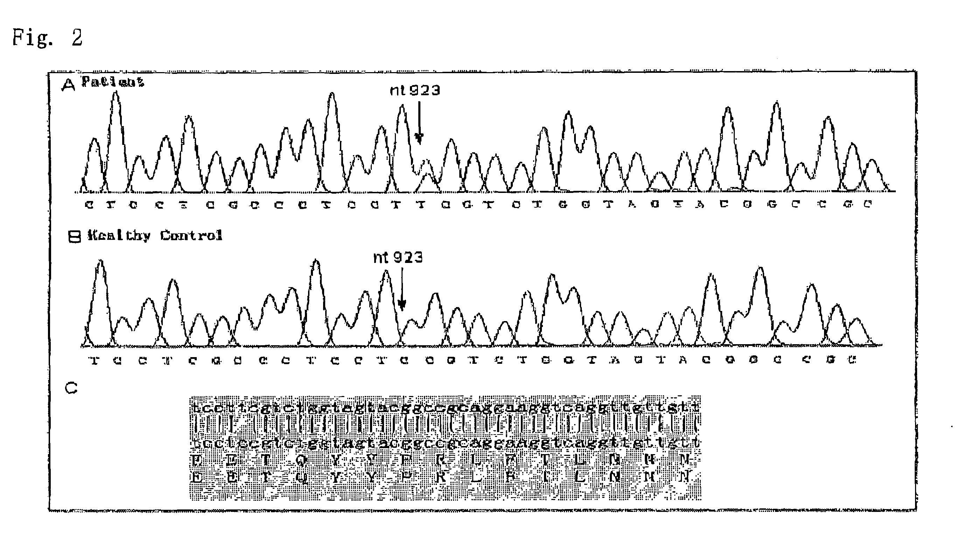

Figure 2 shows the sequence maps of partial DNA containing mutation site G923A (corresponding amino acid mutation E282K) of CACNA1H gene from CAE patient and from healthy control. A: sequence map of partial DNA containing mutation site from CAE patient. B: sequence map of partial DNA from healthy control. C: nucleotide sequence and its corresponding amino acid sequence containing mutation site E282K. Herein nt indicates the position of mutant nucleotide in sequence AF051946.

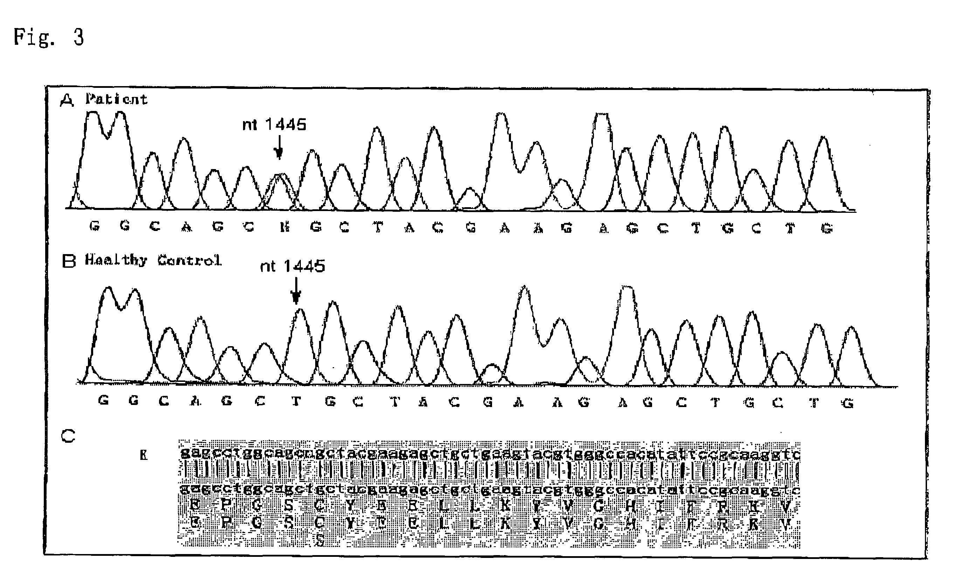

Figure 3 shows the sequence maps of partial DNA containing mutation site T1445A (corresponding amino acid mutation C456S) of CACNA1H gene from CAB patient and from healthy control. A: sequence map of partial DNA containing mutation site from CAE patient. B: sequence map of partial DNA from healthy control. C: nucleotide sequence and its corresponding amino acid sequence containing mutation site C456S. Herein nt indicates the position of mutant nucleotide in sequence AF051946.

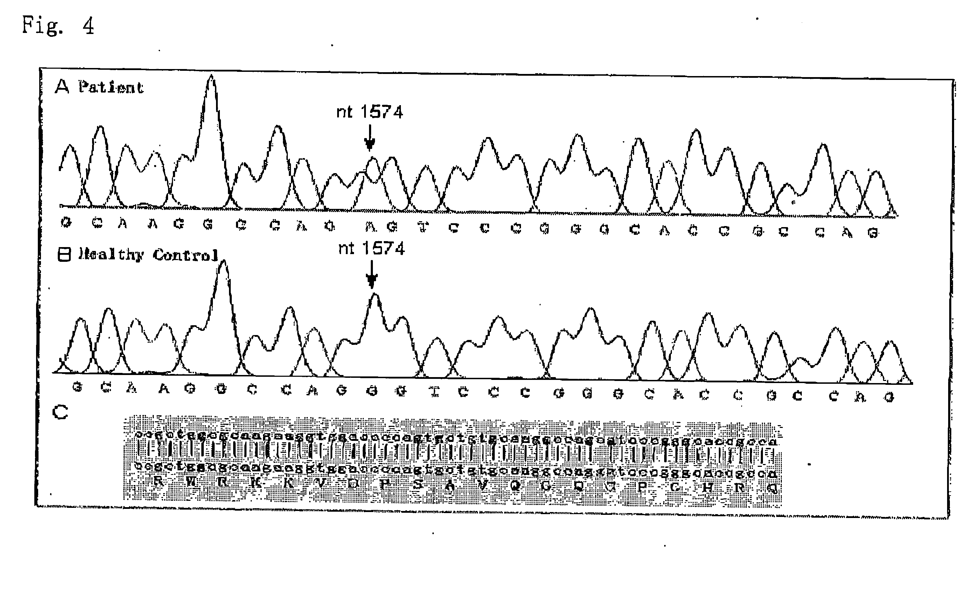

Figure 4 shows the sequence maps of partial DNA containing mutation site G1574A (corresponding amino acid mutation G499S) of CACNA1H gene from CAB patient and from healthy control. A: sequence map of partial DNA containing mutation site from CAE patient. B: sequence map of partial DNA from healthy control. C: nucleotide sequence and its corresponding amino acid sequence containing mutation site G499S. Herein nt indicates the position of mutant nucleotide in sequence AF051946.

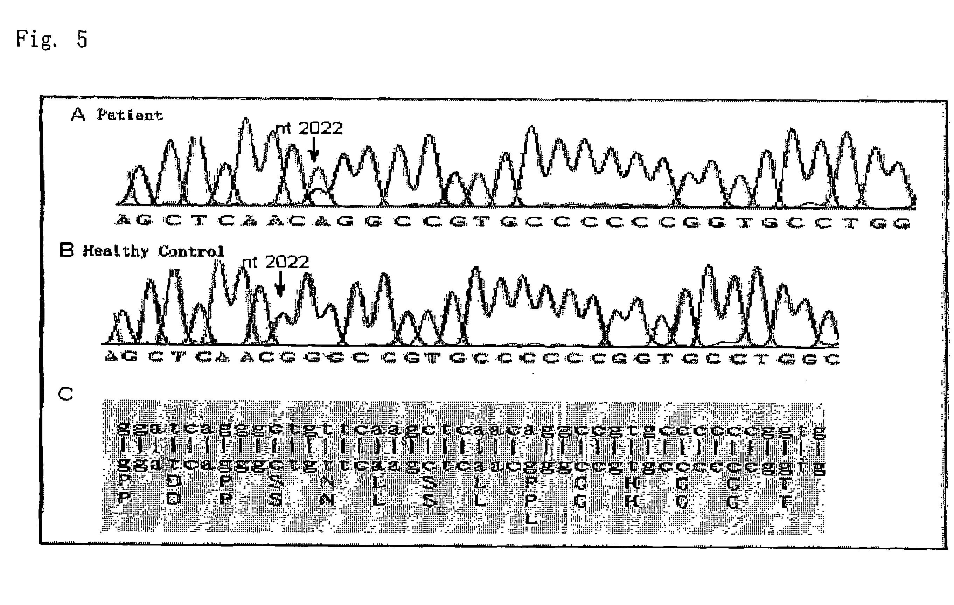

Figure 5 shows the sequence maps of partial DNA containing mutation site C2022T (corresponding amino acid mutation P648L) of CACNA1H gene from CAE patient and from healthy control. A: sequence map of partial DNA containing mutation site from CAB patient B: sequence map of partial DNA from healthy control. C: nucleotide sequence and its corresponding amino acid sequence containing mutation site P648L. Herein nt indicates the position of mutant nucleotide in sequence AF051946.

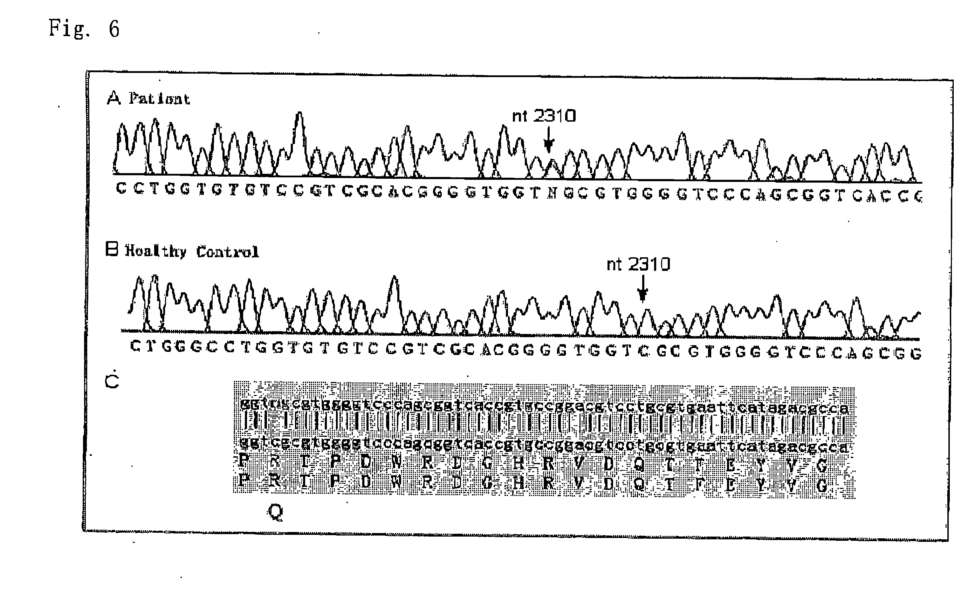

Figure 6 shows the sequence maps of partial DNA containing mutation site G2310A (corresponding amino acid mutation R744Q) of CACNA1H gene from CAE patient and from healthy control. A: sequence map of partial DNA containing mutation site from CAE patient B: sequence map of partial DNA from healthy control. C: nucleotide sequence and its corresponding amino acid sequence containing mutation site R744Q. Herein nt indicates the position of mutant nucleotide in sequence AF051946.

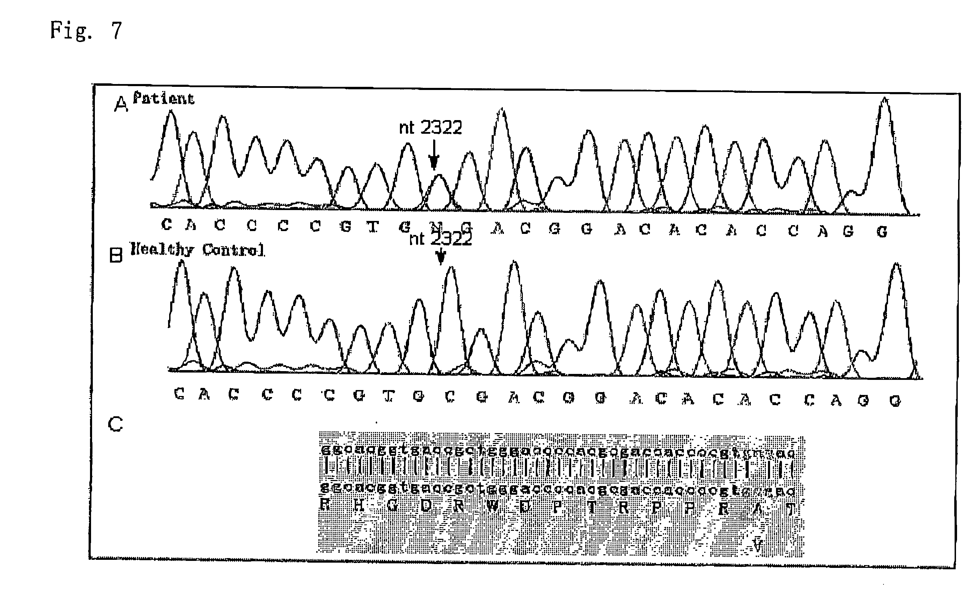

Figure 7 shows the sequence maps of partial DNA containing mutation site C2322T (corresponding amino acid mutation A748V) of CACNA1H gene from CAB patient and from healthy control. A: sequence map of partial DNA containing mutation site from CAE patient B: sequence map of partial DNA from healthy control. C: nucleotide sequence and its corresponding amino acid sequence containing mutation site A748V. Herein nt indicates the position of mutant nucleotide in sequence AF051946.

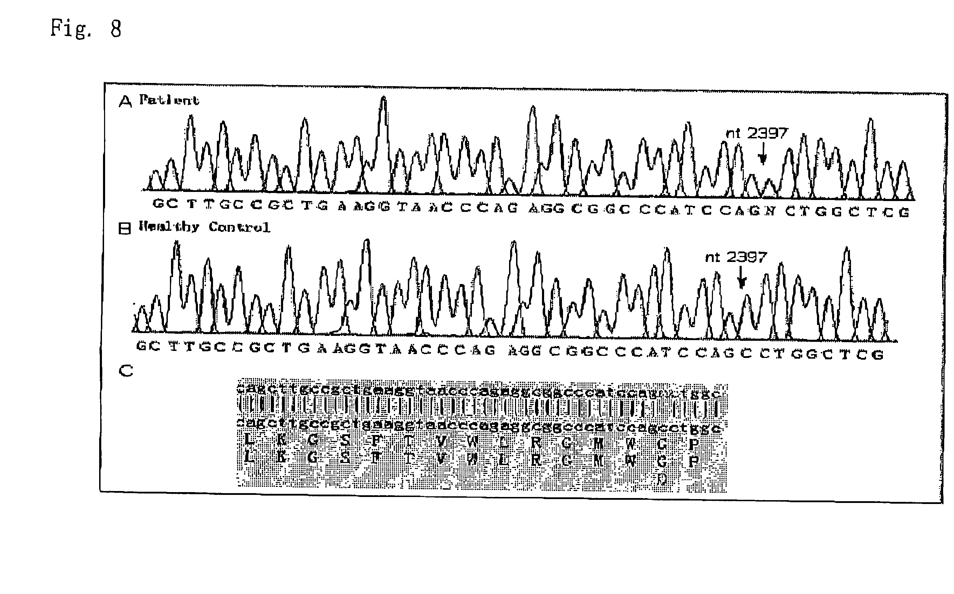

Figure 8 shows the sequence maps of partial DNA containing mutation site G2397A (corresponding amino acid mutation G773D) of CACNA1H gene from CAE patient and from healthy control. A: sequence map of partial DNA containing mutation site from CAE patient B: sequence map of partial DNA from healthy child C: nucleotide sequence and its corresponding amino acid sequence containing mutation site G773D. Herein nt indicates the position of mutant nucleotide in sequence AF051946.

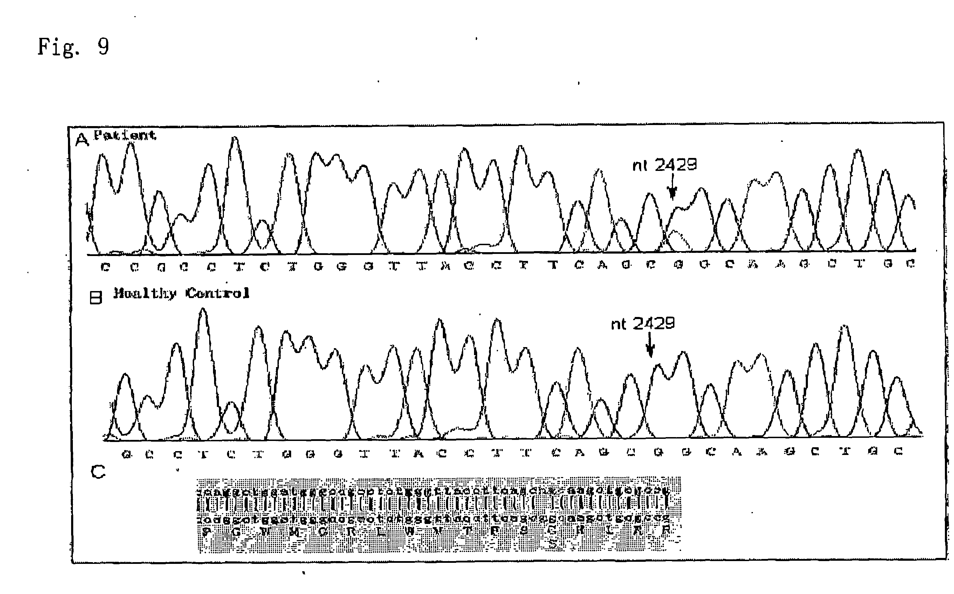

Figure 9 shows the sequence maps of partial DNA containing mutation site G2429A (corresponding amino acid mutation G784S) of CACNA1H gene from CAE patient and from healthy control. A: sequence map of partial DNA containing mutation site from CAE patient. B: sequence map of partial DNA from healthy control. C: nucleotide sequence and its corresponding amino acid sequence containing mutation site G784S. Herein nt indicates the position of mutant nucleotide in sequence AF051946.

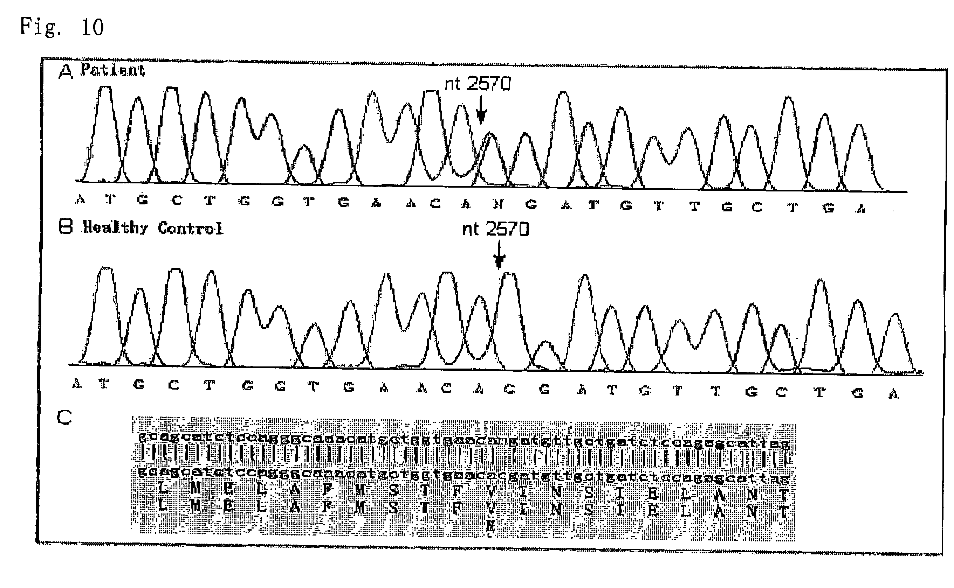

Figure 10 shows the sequence maps of partial DNA containing mutation site G2570A (corresponding amino acid mutation V831M) of CACNA1H gene from CAE patient and from healthy control. A: sequence map of partial DNA containing mutation site from CAE patient B: sequence map of partial DNA from healthy control. C: nucleotide sequence and its corresponding amino acid sequence containing mutation site V831M. Herein nt indicates the position of mutant nucleotide in sequence AF051946.

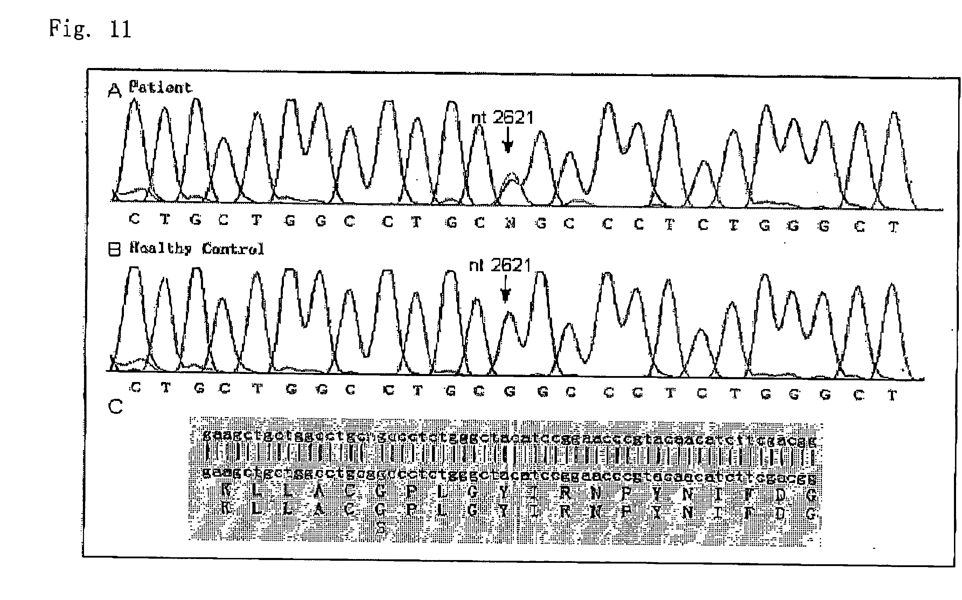

Figure 11 shows the sequence maps of partial DNA containing mutation site G2621A (corresponding amino acid mutation G848S) of CACNA1H gene from CAE patient and from healthy control. A: sequence map of partial DNA containing mutation site from CAE patient. B: sequence map of partial DNA from healthy control. C: nucleotide sequence and its corresponding amino acid sequence containing mutation site G848S. Herein nt indicates the position of mutant nucleotide in sequence AF051946.

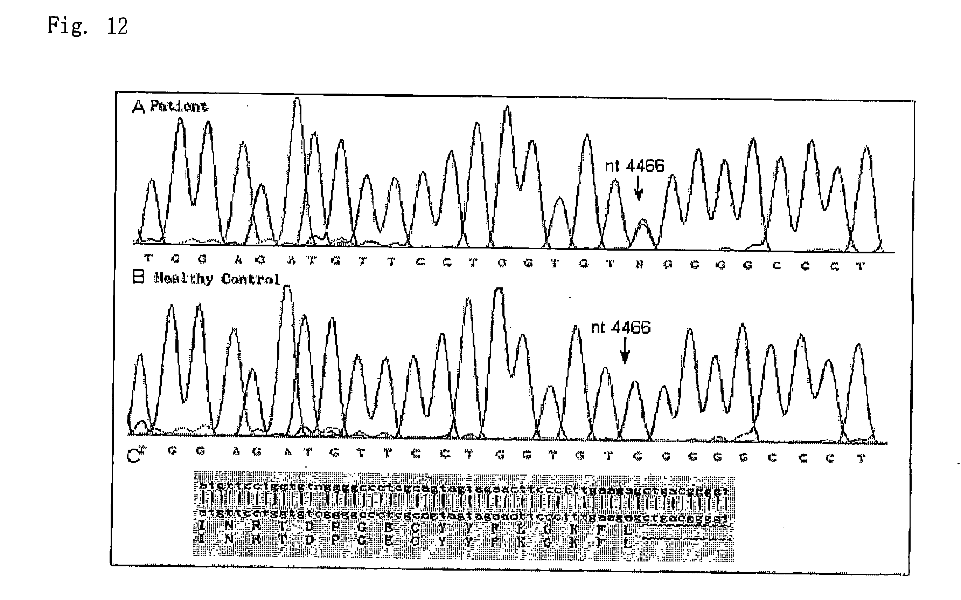

Figure 12 shows the sequence maps of partial DNA containing mutation site G4466A (corresponding amino acid mutation D1463N) of CACNA1H gene from CAE patient and from healthy control. A: sequence map of partial DNA containing mutation site from CAB patient. B: sequence map of partial DNA from healthy control. C: nucleotide sequence and its corresponding amino acid sequence containing mutation site D1463N. Herein nt indicates the position of mutant nucleotide in sequence AF051946.

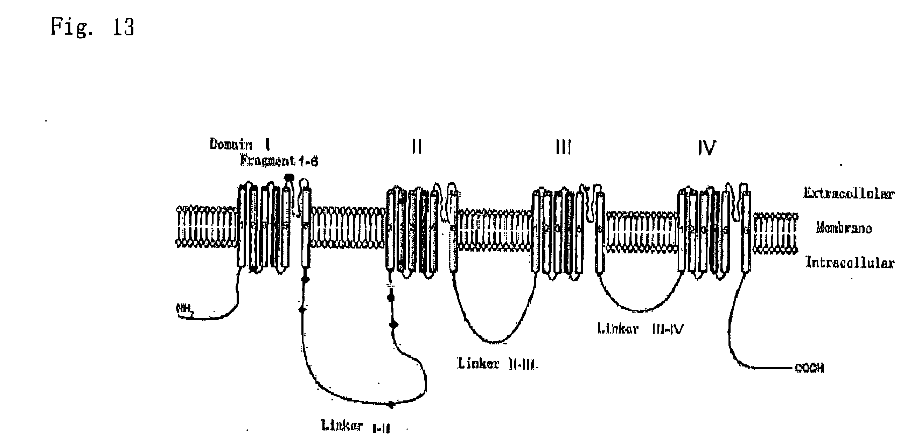

Figure 13 is the schematic diagram of domains of CACNA1H gene, herein mutation sites are shown.

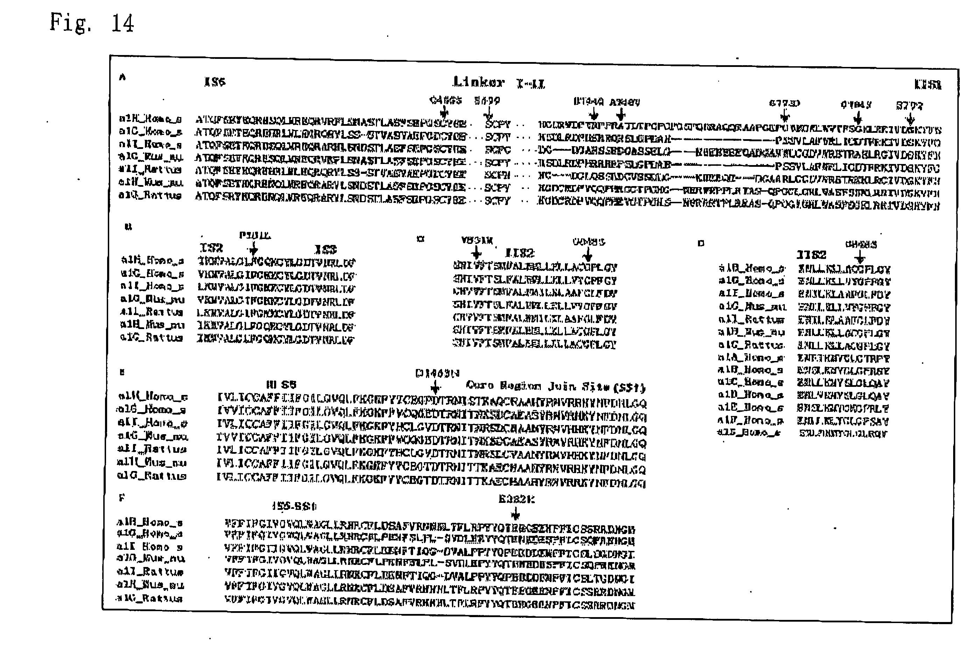

Figure 14 shows the results of research on conservatism of mutation sites. Herein alA- homo-s, alB-homo-s, alC-homo-s, alD-homo-s, alE-homo-s, alF-homo-s, alG-homo-s, alH-homo-s, alI-homo-s, alS-homo-s represent human gene alA, alB,alC,alD,alE,alF, alG, alH, alI, alS gene; alH-Mus-mu, alG-Mus-mu represent mouse alH alG gene; alG-Rattus,alI-Rattus represent rat alG. alI gene.

### EXAMPLES

### EXAMPLE 1

1. PCR amplification of exons of CACNA1H gene
   Object: 118 of CAE patients of north China, Han nationality, coming from out patient and in patient of Beijing university attached First hospital, Beijing Children's hospital and Capital institute of pediatrics respectively. Ages of child patients were years of 2.9-14 with average of 8.5 and the average attack age of 7.2. 230 of healthy person, Han Nationality, coming from the same area of north China were selected as the control. Ages of them were years of 17-23 with average age of 19.5. CAE was diagnosed according to classified diagnose standard of the epilepsy and epileptic syndrome, which was proposed by International Antiepilepsy Association in 1989. The clinic criteria for a CAE patient being selected into case group included (A) Affection at age of 3-12 with absence as the initial symptom. In the course of disease might happen a little general tonoclonic attack and/or myoclonus attack; (B) At the stage of attack the electroencephalogram presented outbreak of bilaterally symmetric synchronous 3Hz rhythmic spike and slow wave, or it could also present as multi-spike and slow wave, usually it was 3Hz in the middle main part and a slight quicker than 3Hz(3.5-4Hz) at the beginning part and a slight slower than 3Hz(about 2.5 Hz) before ending. The background action was normal. The clinic attack and discharge were easily induced at that time of hyperventilation for the patient without being treated ;(C) nervous system examination showed normal; (D) Nervous image (CT or MRI) examination showed normal.
2. DNA extraction: Genomic DNA of leukocyte of peripheral blood was isolated using Miller's modified salting-out method.
   (1) Took 5-15 µl of sodium citrate or EDTANa₂ anticoagulant whole blood (don't use heparin anticoagulant as far as possible) place it into 50ml capped centrifuge tube..
   (2) Added 3-5 volumes of cold distilled H₂O, mixed well by repeat inversion and allow stand on ice for 5 minutes. Centrifuged at 2,000 rpm for 20 minutes at 4°C.
   (3) Slowly poured off the supernatant fluid. To the pellet added pre-cooled 0.1 % triton X-100 (with the same volume as above) and resuspended the pellet by gentle vortex. Centrifuged and discarded the supernatant as described above.
   (4) Added 4 ml of lysis solution (50mM Tris-Cl and 10 mM BDTA, PH 8.0) to the pellet and dispersed it thoroughly. Then added 10% SDS to a final concentration of 0.5% and mixed well.
   (5) Added proteinase K(10mg/ml) to a final concentration of 200ug/ml and digested at 55°C for 3 hrs or at 37°C overnight (overnight was preferred).
   (6) Added 1.2 ml of 6M NaCl, vigorously shook for 20 seconds.
   (7) Centrifuged at 2,200rpm for 10 minutes and transferred the supernatant into another capped centrifuge tube.
   (8) Added two volumes of anhydrated alcohol and repeatedly inverted until appearance of flocculent DNA, picked up DNA to an Eppendorf tube.
   (9) Washed the DNA twice with 75% alcohol.
   (10) Dissolved the DNA in 0.5 ml TE (10 mM Tris-Cl-EDTA pH8.0) or distilled H₂O. Measured the size of DNA fragment by electrophoresis or estimated the ratio of OD₂₆₀/OD₂₈₀.
3. PCR amplification
   Total 32 pairs of primers were designed for 35 exons of CACNA1H gene using software Primer 3 (table 1). A total volume of 15 µl PCR system consisted of 50 ng genomic DNA, 10mM Tris-HCl (pH 8.4), 50 mM KCl, 3.0 mM MgCl₂, 200µM of dNTPs (Amersham Phamacia Product), 0.6U HotStartaq™ DNA polymerase, primers each 0.3 µM. PCR was performed on a Perkin Elmer 9700 themacycler (Applied Biosystems Inc.) under the conditions: in the first 15 cycles, denaturing at 94°C for 30 sec, annealing at 63 °C for 60 sec with the annealing temperature gradually reduced by 0.5°C each cycle, extending at 72°C for 110 sec; in the later 25 cycles, denaturing at 94°C for 30 sec, annealing at 56°C for 60 sec and extending at 72°C for 40 sec; finally, extending at 72°C for 10 minutes.

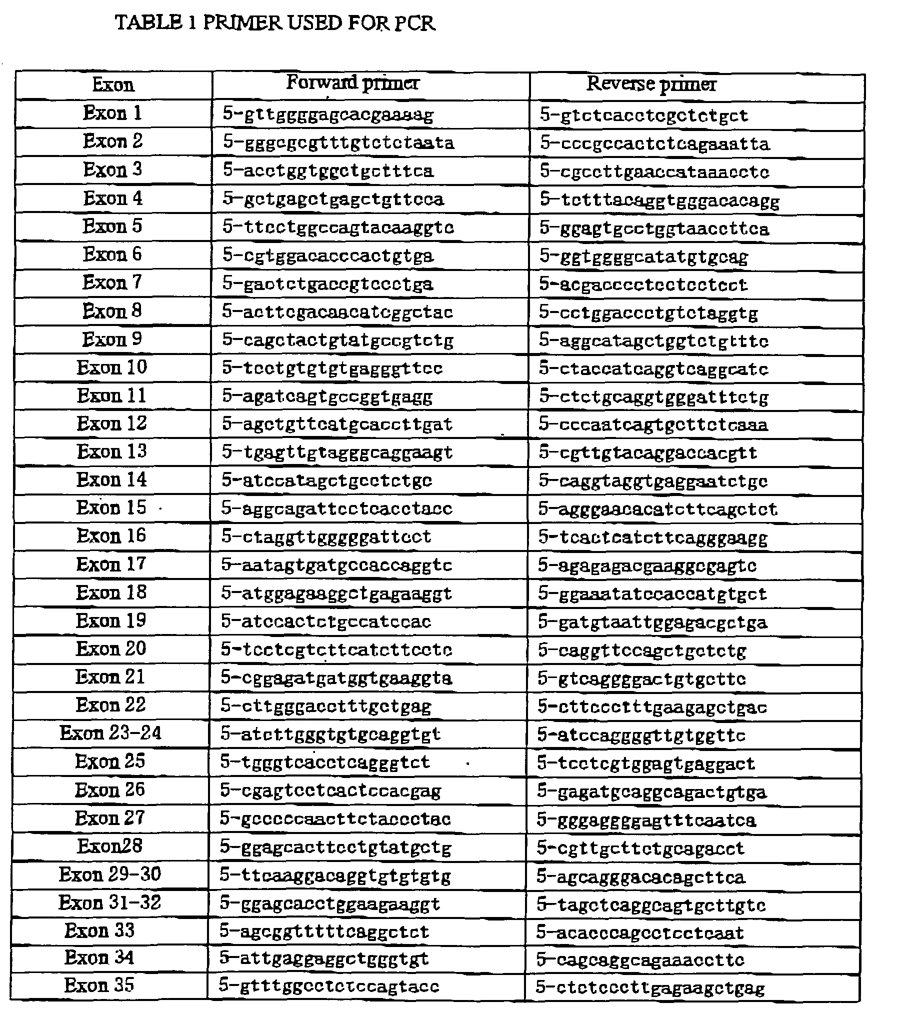

### EXAMPLE 2

### Purification of PCR product.

Took 1µl of PCR product to measure the DNA size by 1%agarose gel electrophoresis and to quantify the DNA roughly. After that the PCR product was purified using 96 well purification plate (Millipore company). The procedure was as follows:
(1) Added 80 µl of deioned H₂O to the 15 µl of first PCR system. Mixed well by shaking and then transferred into a multiscreen-PCR plate.
(2) Placed the Multiscreen-PCR plate on the trestle of vacuum filter for drying under the pressure of 0.485 Mpa for 5-20 minutes. Operated for another 30 seconds after each well was dried up.
(3) Took down the Multiscreen-PCR plate from trestle of vacuum filter and added 15-30 µl deiond H₂O to each well. Allowed stand at room temperature for 30 minutes.
(4) Placed the Multiscreen-PCR plate on mixer and shook for 5 minutes to redissolved the purified products. Transferred them to a new 96 well plate using pipette.
(5) Took 2µl of purified product for quantifying by electrophoresis.

### EXAMPLE 3

### Sequencing reaction and verification

1. Sequencing reaction: Sequencing was carried out on 3700 automatic sequencer (PE Company) using Big-Dye end labeling kit produced by PE Company with 2 µl of purified product as template and one side primer (diluted to 2 µM.) of the pair of PCR primers as sequencing primer.
In a total volume of 10 µl reaction system the following components as shown in table 2 were contained.

**TABLE 2**

| REACTION SYSTEM | | | |
|---|---|---|---|
| Component | Conc.of stock solution | Amount added (µl) | Final conc. |
| Buffer | 5× | 2 | 1× |
| Primer | 2µM | 2 | 4 pmol |
| Big-Dye mix | | 1.2 | |
| Template | | 2 | 3-10 ng |
| ddH₂O was added to bring up the final volume to 10 µl and then sequencing reaction was performed on Perking Elmer 9700 thermocycler (Applied Biosystems Inc.) | | | |

2. Purification of product of sequencing reaction and determination of the sequence: The product of sequencing reaction was recipitafed by alcohol and the nucleotide sequence was determined on ABI337 or 3700 automatic sequencer (Applied Biosystems Inc.)
3. The mutation was further defined on the protein level after translating according to normal reading frame. It included the following step: translation was done according to normal reading frame whereby the mutation site could be defined

### Analysis of results

The sequencing map obtained was analyzed The appearance of hybrid double peaks at any sites as shown in figure I to figure 12 indicated the hybrid states of these sites and further verifications were needed. First, sequencing reaction was performed again but using opposite another side primer of the pair of PCR primers as sequencing primer. If the result confirmed that it was really a mutation site, then the sequencing of DNA fragment containing this site, taken from the parents of the child patient is performed. Our results have showed that all the mutations found could be appeared in one of the parent, so they were the hybrid mutations. Afterwards the results of sequencing reaction of CAGNA1H gene taken from the parents of 230 healthy controls indicated that no any mutation sites could be found

The direct DNA sequencing of 35 exons of CACNA1H genes derived from 118 of child patients, Han nationality, of north China were conducted. By a large number of experiments and statistical analysis of a great quantity of specimens, 12 different sites occurring missense mutation were found from 14 CAE sufferers. The exons in which the mutation sites were located, the replacement of nucleotides and amino acids as well as structural localization of these sites are shown in table 3. The Schematic drawing of domains is shown in Figure 13.

**TABLE 3**

| Patient | Exon | Replacement of nucleotide | Replacement of amino acid | Structural localization |
|---|---|---|---|---|
| 1 | 4 | C562A | F161L | IS2-IS3 |
| 2 | 7 | G923A | E282K | IS5-SS1 |
| 3 | 9 | T1445A | C456S | Linker I-II |
| 4 | 9 | G1574A | G499S | Linker I-II |
| 5 | 9 | C2022T | P648L | Linker I-II |
| 6 | 10 | G2310A | R744Q | Linker I-II |
| 7 | 10 | G2310A | R744Q | Linker I-II |
| 8 | 10 | C2322T | A748V | Linker I-II |
| 9 | 10 | G2397A | G773D | Linker I-II |
| 10 | 10 | G2397A | G773D | Linker I-II |
| 11 | 10 | G2429A | G784S | Linker I-II |
| 12 | 11 | G2570A | V831M | IIS2 |
| 13 | 1 | G2621A | G848S | IIS2 |
| 14 | 23 | G4466A | D1463N | (IIIS5-SS1) |
| Note: The positions of the mutation nucleotides and the changes of corresponding amino acids thereof were numbered based on Genbank data (Accession number AF051946.1) | | | | |

### EXAMPLE 4

### Research on conservatism of mutation sites

Comparing the amino acid sequence of three T type channel genes (CACNAIG, CACNA1H and CACNAII) of human with that of two T type channel genes of mouse and rat each, we found the mutation F161L locating at transmembrane region 2 and 3 of domain I (IS2-IS3), the mutation E282K locating at the linker of transmembrane region S5 of domain I and core region (IS5-SS 1), the mutation D1463N locating at the linker of transmembrane region S5 of domain III and core region (IIIS5-SS1), the mutations V831M and G848S both locating at transmembrane region II (IIS2) and the mutation C456S locating at the linker of domain I and domain II (linkerI-II) were all localized at highly conservative amino acid sites of the seven T-type Calcium channel genes. Furthermore the mutation site G848S at transmembrane region 2 of domain II (II -S2) is also highly conservative in the encoding products of seven other human high voltage-activated Calcium channel genes.

### EXAMPLE 5

The kit for detecting mutation of CACNA1H gene, one of the major genes for CAE and its application.
1. The kit contained:
   Primers used for amplification 
Exon | Forward primer | Reverse primer
Exon 1 | 5-gttggggagcacgaaaag | 5-gtctcacctcgctctgct
Exon 2 | 5-gggcgcgtttgtctctaata | 5-cccgccactctcagaatta
Exon 3 | 5-acctggtggctgctttca | 5-cgccttgaaccataaacctc
Exon 4 | 5-gctgagctgagctgttcca | 5-tctttacaggtggacacagg
 
Exon 5 | 5-ttcctggccagtacaaggtc | 5-ggagtgcctggtaaccttca
Exon 6 | 5-cgtggacacccactgtga | 5-ggtggggcatatgtgcag
Exon 7 | 5-gactctgaccgtccctga | 5-acgacccctcctcctcct
Exon 8 | 5-acttcgacaacatcggctac | 5-cctggaccctgtctaggtg
Exon 9 | 5-cagctactgtatgccgtctg | 5-aggcatagctggtctgtttc
Exon 10 | 5-tcctgtgtgtgagggttcc | 5-ctaccatcaggtcaggcatc
Exon 11 | 5-agatcagtgccggtgagg | 5-ctctgcaggtgggatttctg
Exon 12 | 5-agctgttcatgcaccttgat | 5-cccaatcagtgcttctcaaa
Exon 13 | 5-tgagttgtagggcaggaagt | 5-cgttgtacaggaccacgtt
Exon 14 | 5-atccatagctgcctctgc | 5-caggtaggtgaggaatctgc
Exon 15 | 5-aggcagattcctcacctacc | 5-agggaacacatcttcagctct
Exon 16 | 5-ctaggttggggattcct | 5-tcactcatcttcagggaagg
Exon 17 | 5-aatagtgatgccaccaggtc | 5-agagagacgaaggcgagtc
Exon 18 | 5-atggagaaggctgagaaggt | 5-ggaaatatccaccatgtgct
Exon 19 | 5-atccactctgccatccac | 5-gatgtaattggagacgctga
Exon 20 | 5-tcctcgtcttcatcttcctc | 5-caggttccagctgctctg
Exon 21 | 5-cggagatgatggtgaaggta | 5-gtcaggggactgtgcttc
Exon 22 | 5-cttgggacctttgctgag | 5-cttccctttgaagagctgac
Exon 23-24 | 5-atcttgggtgtgcaggtgt | 5-atccaggggttgtggttc
Exon 25 | 5-tgggtcacctcagggtct | 5-tcctcgtggagtgaggact
Exon 26 | 5-cgagtcctcactccacgag | 5-gagatgcaggcagactgtga
Exon 27 | 5-gcccccaacttctaccctac | 5-gggaggggagtttcaatca
Exon 28 | 5-ggagcacttcctgtatgctg | 5-cgttgcttctgcagacct
Exon 29-30 | 5-ttcaaggacaggtgtgtgtg | 5-agcagggacacagcttca
Exon 31-32 | 5-ggagcacctggaagaaggt | 5-tagctcaggcagtgcttgtc
Exon 33 | 5-agcggtttttcaggctct | 5-acacccagcctcctcaat
Exon 34 | 5-attgaggaggctgggtgt | 5-cagcaggcagaaaccttc
Exon 35 | 5-gtttggcctctccagtacc | 5-ctctcccttgagaagctgag
 Hotstar Taq DNA polymerase 5U / µl
      10 x buffer (containing 15 mM MgCl₂)
      dNTP 2 mM
      Big-Dye mix
2. Method for use
   Mainly the following steps were included
   A: Isolated DNA and conducted PCR amplification using primers described above;
   B: After purification of PCR product, determined the sequence directly. Then compared the sequence obtained with that in accession number AF 151946.1 of Genbank so as to define the mutation site in CACNA1H gene;
      Further step included C: Translated the nucleotide sequence according to normal reading frame thereby to define the mutation site in CACNA1H gene.
      For the detailed procedure see example 1,2 and 3.

### EXAMPLE 6

The kit for detecting mutation site F161L of CACNA1H, one of the major genes for CAB and its application.
1. The kit contained:
   P rimers for amplifying exon4: Forward primer: 5-gctgagctgagctgttcca Reverse primer: 5-tctttacaggtgggacacagg
   Hotstar Taq DNA polymerase 5U/µl
      10x buffer (containing 15 mM MgCl₂)
      dNTP 2 mM
   Big-dye mix
2. Method for use
   Mainly the following steps were included:
   A: Isolated DNA and conducted PCR amplification using above primers;
   B: After purification of PCR product, determined the sequence directly. Then compared the sequence obtained with that in accession number AF151946.1 of Grenbank so as to define the mutation site in CACNA1H gene;

Furthermore step included C: translated the nucleotide sequence according to normal reading frame thereby to define the mutation site in CACNA1H gene.

### EXAMPLE 7

The kit for detecting mutation site E282K of CACNA1H, one of the major genes for CAE and its application.
1.The kit contained;
   Primers for amplifying the DNA fragment containing mutation site E282K Forward primer. 5-gggaagttctactactgcgagggcccctatagacaccagg Reverse primer: 5-ctgtgcgcacctgctggtcgacacccacggcatccagcccg Hotstar Taq DNA polymeryase 5 U/l
   10x buffer (containing 15 mM MgCl₂)
   dNTP 2 mM
   Restriction enzyme EcoRV
   Restriction map
2. Method for use
   A: Isolated DNA and PCR amplified using above primers;
   B: After purification of PCR product, deternnined the sequence directly. Then compared the sequence obtained with that in accession number AF151946.1 of Genbank so as to define the mutation site in CACNA1H gene;
   C: Digested the PCR product by EcoRV;
   D: Compared the restriction map obtained above with that given in kit.

It is known to the general skilled in the art, for some components in the above kit, such as primers, restriction enzymes etc., the corresponding alterations can be made according to the different sites to be detected. The technician should amplify the sample of blood DNA from patient by PCR using the method mentioned above, then digest the PCR product and compare the restriction map with that given in the kit.

It should understand that after reading the contents of the present invention described above the skilled in the art can make various alterations or modifications. But any of all equal value forms of alterations or modifications would fall into the scope defined by the claims of the present application.

## Claims

1. A method for detecting the CACNA1H mutant gene, one of the major genes for CAE, wherein said method is direct DNA sequencing or restriction digestion.

2. The method for detecting the CACNA1H mutant gene according to claim 1, wherein at least one mutation site of said CACNA1H mutant gene is located at the site selected from the group consisting of the transmembrane region 2 and 3 of domain I, the join site of transmembrane region S5 of domain I and core region, the transmembrane region 2 of domain II, the linker of domain I and II or the join site of transmembrane region S5 of domain III and core region.

3. The method for detecting the CACNA1H mutant gene according to claim 2, wherein at least one mutation site of said CACNA1H mutant genes is locatedatthe site selected from the group consisting of C562A, G923A, T1445A, G1574A, C2022T, G2310A, G2397A, G2429A, G2570A, G2621A and G4466A.

4. The method of detecting the CACNA1H mutant gene described according to claim 3, wherein at least one mutation site of said CACNA1H mutant gene is located at the site selected from the group consisting of C562A, G923A, G2570A, G2621A, T1445A and G4466A.

5. The method for detecting the CACNA1H mutant gene according to any of claims 1-4, **characterized in that** said method includes the following steps:
A: Design PCR primer aiming at 35 exons of CACNA1H gene and amplify by PCR;
B: Determine the sequence of purified PCR product directly and compare the determined sequence with that in Genbank thereby to define the mutation site in CACNA1H gene.

6. The method for detecting the CACNA1H mutant gene according to claim 5, **characterized in that** said method further include the following step:
C: Translate the nucleotide sequence according to normal reading frame thereby to define the mutation site in CACNA1H gene.

7. The method for detecting the CACNA 1H mutant gene according to any of claim 1-4, **characterized in that** said method includes the following steps:
A: Isolate DNA and design PCR primers against the area containing the mutation site and amplify by PCR;
B: Determine the sequence of purified PCR product directly and compare the determined sequence with that in Genbank thereby to define the mutation site in CACNA1H gene.

8. The method for detecting the CACNA1H mutant gene according to claim 7, **characterized in that** said method further includes the following step:
C: Translate the nucleotide sequence according to normal reading frame thereby to define the mutation site in CACNA1H gene.

9. A CACNA1H mutant gene, one of the major genes for CAE, it contains the sequence of T-type Calcium channel gene CACNA1H and at least one mutation site in this sequence is located at the site selected from the group consisting of transmembrane region 2 and 3 of domain I, the join site of transmembrane region S5 of domain I and core region, the transmembrane region 2 of domain II, the linker of domain I and II and the join site of transmembrane region S5 of domain III and core region.

10. The CACNA1H mutant gene according to claim 9, wherein at least one mutation site of said CACNA1H sequence is located at the site selected from the group consisting of C562A, G923A, T1445A, G1574A, C2022T, G2310A, C2322T, G2397A, G2429A, G2570A, G2621A and G4466A.

11. The CACNA1H gene according to claim10, wherein at least one mutation site of said CACNA1H sequence is located at the site selected from the group consisting of C562A, G923A, G2570A, G2621A, T1445A and G4466A.

12. A kit for detecting the CACNA1H mutant gene, one of the major genes for CAE, wherein said kit contains primers for amplification, dTNP, one or several kinds of DNA polymerase and buffer solution thereof used for PCR.

13. A kit for detecting the CACNA1H mutant gene, one of the major genes for CAE, wherein said kit contains:
1) Primers for amplifying the mutation sites;
2) Enzyme for amplification by PCR and its corresponding buffer solutions;
3) The restriction enzymes for digestions of different mutation sites;
4) The restriction maps produced by digesting different mutation sites.

14. Application of the kit according to claim12 or13 in examination and/or therapy method of CAE.

15. Application of the method for detecting CACNA1H mutant gene according to any of claim 1-8 in examination and/or therapy method of CAE.

16. Application of the mutant genes according to any of claim 9-11 in examination and/or therapy method of CAE.
